# EUROPEAN PATENT APPLICATION

(11) **EP 4 310 187 A1**
(43) Date of publication of application: **24.01.2024**
(21) Application number: 22771428.4
(22) Date of filing: 15.03.2022
(51) Int. Cl.: C12N 15/62, A61K 38/16, A61K 47/64, A61P 1/16, A61P 9/00, A61P 9/10, A61P 11/00, A61P 11/06, A61P 13/12, A61P 21/00, A61P 25/00, A61P 25/02, A61P 25/16, A61P 25/28, A61P 29/00, A61P 37/02, A61P 39/06, C07K 14/435, C07K 14/765, C07K 19/00

(54) **MODIFIED HUMAN SERUM ALBUMIN-THIOREDOXIN FUSION BODY**

(30) Priority: 16.03.2021 JP 2021042220
(71) Applicant: KM Biologics Co., Ltd., Kumamoto-shi, Kumamoto 860-8568 (JP); National University Corporation Kumamoto University, Kumamoto 860-8555 (JP)
(72) Inventor: MATSUDA, Junichi, Kikuchi-shi, Kumamoto 869-1298 (JP); NISHIDA, Kento, Kikuchi-shi, Kumamoto 869-1298 (JP); HIRASHIMA, Masaki, Kikuchi-shi, Kumamoto 869-1298 (JP); MARUYAMA, Toru, Kumamoto-shi, Kumamoto 860-8555 (JP); WATANABE, Hiroshi, Kumamoto-shi, Kumamoto 860-8555 (JP); MAEDA, Hitoshi, Kumamoto-shi, Kumamoto 860-8555 (JP)
(74) Representative: Bandpay & Greuter
(86) International application number: PCT/JP2022/011580
(87) International publication number: WO 2022/196683

(57) **Abstract**

Disclosed is a serum albumin-thioredoxin fusion body which is improved in the activity thereof and is stable with respect to the activity. The serum albumin-thioredoxin fusion body is provided, which is characterized in that the thioredoxin is a modified from in which at least a cysteine residue located at position 73 from the N-terminal of an amino acid sequence for the thioredoxin or located at a position equivalent to the position 73 is substituted by another amino acid residue. the modified serum albumin-thioredoxin fusion body is superior in the activity and stability thereof, is reduced in immunogenicity and has superior safety compared with a fusion body in which the thioredoxin is non-modified form.

## Description

### [Technical Field]

The present invention relates to a modified form of a human serum albumin-thioredoxin fusion body, more specifically to a fusion body having a thioredoxin activity as well as stability and safety of its pharmacological action improved by a modification.

### [Background Art]

Thioredoxin (Trx) was discovered in 1964 as a coenzyme of a ribonucleotide reduction enzyme of E. Coli (NPL 1). A human Trx was described by Yodoi et al., as a result of gene cloning in the late 1980s, to be a homologue of an adult T-cell leukemia-derived factor (ATL-derived factor: ADF) that was reported as an inducing factor of the interleukin-2 receptor alpha-chain (NPL 2).

The Trx is coded as a protein having the molecular weight of about 14,000 in the TXN gene of the human 9th chromosome, and expresses in various cells in all of biological species. In the case of human, the loss-of-function mutant of the Trx gene is lethal at the four-cell stage in the early period of embryo development. The Trx has an active site with -Cys-Gly-Pro-Cys- and is induced by an oxidation stress originated from various causes such as UV lights, radioactive rays, oxidants, virus infections, ischemia-reperfusion injury, and anti-cancer therapy, then exhibits an anti-oxidation activity by eliminating active oxygen species in a cell (NPLs 1 and 3). In addition, this has pharmacological activities such as activity control of cytokine and of transcription factors such as a nuclear factor-κB (NF-κB) and an apoptotic signal-regulating kinase-1 (ASK-1), an anti-apoptotic action, and an anti-inflammatory action to suppress an inflammatory cytokine and a human macrophage migration inhibitory factor (MIF) (NPLs 4 to 7).

The human serum albumin (HSA) is a protein having a molecular weight of 66,000 that is synthesized in liver, occupying about 50 to 65% of serum proteins. The HSA has functions such as keeping blood's osmotic pressure, pH buffering, feeding amino acids to various tissues, and as an anti-oxidant, as well as functions to bond with foreign substances such as fatty acids, bilirubin, inorganic ions, and chemicals.

Since the half-life of the HSA in blood is 14 to 21 days, and a low-molecular weight compound bonded to the HSA is not incorporated into organs, it can circulate in blood without being metabolized and discharged. Although the Trx has various pharmacological effects as described above, the half-life thereof in blood is so short as about 1 hour because it is promptly subject to a glomerulus filtration upon administered as a drug, so that in order to obtain treatment effects with keeping the concentration in blood, a constant rate intravenous administration or a frequent administration is required.

Some of the inventors of the present invention have reported that with use of the albumin fusion technology (PTL 1) where the Trx was fused with HSA (HSA-Trx) the half-life thereof in blood could be changed from 1 hour to 10 hours, resulting in successfully prolonging the residence time thereof in blood (NPL 8). It was reported that this HSA-Trx exhibited its effectiveness in various disease models. For example, it was reported that this exhibited favorable treatment effects by single or low-frequent administration in disease models such as cisplatin nephropathy (NPL 9), acetoaminophenone-induced liver damage (NPL 10), bleomycin-induced pulmonary fibrosis (NPL 11), and influenza-induced lung damage (NPL 12).

The Trx is the protein composed of 105 amino acid residues, and has the structure called a thioredoxin fold, which has both α helix and β sheet commonly contained in the enzymes that catalyze formation of a disulfide (S-S) bond and isomerization. Furthermore, the Trx has the structure in which 4 β sheets are interposed between 2 α helixes.

The Trx has 5 cysteine residues, in which all of them are free thiols (SH). Cys 32 and Cys 35, which are located at positions 32 and 35 from the N-terminal, are the center of the oxidation-reduction activity of the Trx and also the control site of the ASK-1 bonding. Rest of the thiols, Cys 62, Cys 69, and Cys 73, at positions 62, 69, and 73 from the N-terminal, are the site to serve for an allosteric effect, and Cys 69 is the site for nitrosylation; and there is a bonding site with a thioredoxin reductase (TrxR) near Cys 62 and Cys 69, in which the TrxR cannot bond if Cys 62 and Cys 69 are disulfide-bonded. Cys 73 is the site of glutathione addition, and also the site of dimerization of the Trx (Non-Patent Literature 13). It is reported that the modified body in which Cys 32 and Cys 35 are substituted by serine exhibits rapid migration into a cell, thereby having a high suppressive activity of cell proliferation (PTL 2). It is also reported that the substitution of Cys 62, Cys 69, and Cys 73 with serine decreases a Trx activity (reductive activity) as compared with the wild type (NPL 14). There is a report that when the cysteine residue at the activity center of the Trx was not modified, but at least one or all of the rest of the cysteine residues were substituted by another amino acid, this became stable under non-reductive conditions (PTL 3).

On the other hand, the HSA is a simple protein having 585 amino acid residues, composed of homologue regions in which 3 domains, i.e., domain I (1 to 195 residues), domain II (196 to 383 residues), and domain III, are folded; and in addition, each domain has subdomains A and B, among which IIA (Site I) and IIIA (Site II) are sites to bond with many chemicals, and there is a metal-bonding site in the N-terminal side. There are 17 pairs of disulfide (S-S) bond in tis molecule, in which only the 34th cysteine residue (Cys 34) from the N-terminal has a free thiol group (SH). The HSA having this free thiol remained in the free state is called a reduced type HSA, and the HSA having a disulfide formed is called an oxidized type HSA; the oxidized type HSA is used as the marker for various diseases. Furthermore, Zhao et al. reported that in the fused protein (IFN-alpha 2b-HSA) between an interferon-alpha 2b (IFN-alpha 2b) and the HSA, when Cys 34 in the HSA was substituted by serine, the formation of aggregate could be suppressed and the heat-stability thereof could be improved (NPL 15).

### [Citation List]

### [Patent Literatures]

PTL 1: EP0399666A1
PTL 2: WO2004/087917A1
PTL 3: JPH10-191977A

### [Non-Patent Literatures]

NPL 1: Holmgren A, Thioredoxin. Ann Rev Biochem 54 (1985) 237-271
NPL 2: Tagaya Y, et al., ATL-derived factor (ADF), an IL-2 receptor/Tac inducer homologous to thioredoxin; possible involvement of dithiol-reduction in the IL-2 receptor induction. EMBO J 8 (1989) 757-764
NPL 3: Nakamura H, et al., Redox regulation of cellular activation. Annu Rev Immunol 15 (1997) 351-369
NPL 4: Chen B, et al., Thioredoxin 1 downregulates MCP-1 secretion and expression in human endothelial cells by suppressing nuclear translocation of activator protein 1 and redox factor-1. Am J Physiol Cell Physiol 298 (2010) C1170-C1179
NPL 5: Ohashi S, et al., Overexpression of redox-active protein thioredoxin-1 prevents development of chronic pancreatitis in mice. Antioxid Redox Signal 8 (2006) 1835-1845
NPL 6: Yoshioka J, et al., Role of thioredoxin in cell growth from interactions with signaling molecules. Antioxid Redox Signal 8 (2006) 2143- 2151
NPL 7: Son A, et al., Direct association of thioredoxin-1 (TRX) with macrophage migration inhibitory factor (MIF) : regulatory role of TRX on MIF internalization and signaling. Antioxid Redox Signal 11 (2009) 2595-2605
NPL 8: Ikuta S, et al., Albumin fusion of thioredoxin - The production and evaluation of its biological activity for potential therapeutic applications. J Control Release 147 (2010) 17-23
NPL 9: Kodama A, et al., Albumin fusion renders thioredoxin an effective anti-oxidative and anti-inflammatory agent for preventing cisplatin-induced nephrotoxicity. Biochem Biophys Acta 1840 (2014) 1152-1162
NPL 10: Tanaka R, et al., Albumin fusion prolongs the antioxidant and anti-inflammatory activities of thioredoxin in mice with acetaminophen-induced hepatitis. Mol Pharm 11 (2014) 1228-1238
NPL 11: Tanaka R, et al., Long-Acting Human Serum Albumin-Thioredoxin Fusion Protein Suppresses Bleomycin-Induced Pulmonary Fibrosis Progression. J Pharmacol Exp Ther 345 (2013) 271-283
NPL 12: Tanaka R, et al., Therapeutic impact of human serum albumin-thioredoxin fusion protein on influenza virus-induced lung injury mice Front Immunol 5 (2014) 561
NPL 13: Jones DP, Radical-free biology of oxidative stress. Am J Physiol Cell Physiol 295 (2008) C849-C868
NPL 14: Go Y-M, et al., Reactive aldehyde modification of thioredoxin-1 activates early steps of inflammation and cell adhesion Am J Pathol 171 (2007) 1670-1681
NPL 15: Zhao HL, et al., Elimination of the free sulfhydryl group in the human serum albumin (HSA) moiety of human interferon-alpha2b and HSA fusion protein increases its stability against mechanical and thermal stresses. Eur J Pharm Biopharm 72 (2009) 405-411

### [Summary of the Invention]

### [Technical Problem]

According to findings by the inventors of the present invention, it was observed that the serum albumin-thioredoxin fusion body (non-modified type) could not have stable pharmacological effects, resulting in variance in quality and safety, which are unfavorable as a pharmaceutical drug.

The inventors of the present invention have found that when at least cysteine at position 73 from the N-terminal of the sequence of thioredoxin of the serum albumin-thioredoxin fusion body is substituted by another amino acid, the activity of the thioredoxin cab be significantly increased, and also stable pharmaceutical effects could be obtained. In addition, we have found that an immunogenicity thereof can be reduced, so that the safety thereof can be made excellent. The present invention is based on these findings.

Accordingly, an object of the present invention is to provide a serum albumin-thioredoxin fusion body having an activity thereof increased and stabilized. In addition, another object of the present invention is to provide a serum albumin-thioredoxin fusion body having an excellent safety.

### [Solution to Problem]

The present invention is related to a modified serum albumin-thioredoxin fusion body, wherein the thioredoxin is a modified form in which at least cysteine at position 73 from N-terminal of an amino acid sequence thereof or located at a position equivalent thereto is substituted by another amino acid.

### [Brief Description of the Drawings]

[Fig. 1] is the measurement results of the activities of the modified bodies, in which the cysteines at respective sequence positions in the HSA-Trx are substituted by an alanine, relative to the activity of rTrx.
[Fig. 2] is the measurement results of the activities of the modified bodies including substitution of C73 with another amino acid relative to the activity of the non-modified body (HSA-Trx: HT).
[Fig. 3] is the SDS-PAGE electrophoregram of the modified bodies.
[Fig. 4] is the SDS-PAGE electrophoregram of the modified bodies in which the thiol groups in the HSA-Trx, except for cysteines at positions 32 and 35 from the N-terminal of the Trx sequence in the HSA-Trx, are substituted by serine and glycine.
[Fig. 5] is the measurement results of the activities of the modified bodies, in which the thiol groups in the HSA-Trx, except for cysteines at positions 32 and 35 from the N-terminal of the Trx sequence in the HSA-Trx, are substituted by serine and glycine, relative to the activity of the non-modified body (HSA-Trx: HT).
[Fig. 6] is the evaluation results of the anaphylactic reaction and antibody induction ability upon frequent administration of the modified and non-modified body.
[Fig. 7] is the alanine aminotransferase values at 12 hours after administration of various modified bodies to the Concanavalin A-induced acute hepatitis model.
[Fig. 8] is the alanine aminotransferase values at 12 hours after administration of the modified and non-modified body to the concanavalin A-induced acute hepatitis model.
[Fig. 9] is the blood urea nitrogen values at 96 hours after administration of the modified and non-modified body to the cisplatin-induced acute kidney injury model.
[Fig. 10] is the blood urea nitrogen values at 24 hours after administration of the modified and non-modified body to the ischemia reperfusion-induced acute kidney injury model.
[Fig. 11] is the left lung weight at 18 days after administration of the modified and non-modified body to the pulmonary fibrosis-induced model.
[Fig. 12] is the left lung hydroxy proline amount at 18 days after administration of the modified and non-modified body after induction of the pulmonary fibrosis.

### [Description of the Embodiments]

### Definitions and Abbreviations

In this disclosure, following abbreviations are used with the following definitions.
Thioredoxin: Trx
Human serum albumin: HSA
Modified human serum albumin-thioredoxin fusion body: modified HSA-Trx, or simply HSA-Trx
Non-modified human serum albumin-thioredoxin fusion body: non-modified HSA-Trx, or HT
Cysteines at positions 62, 69 and 73 from the N-terminal of the amino acid sequence of thioredoxin: C62, C69, and C73, or Cys 62, Cys 69, and Cys 73.
Modified body in which cysteine at position 34 from the N-terminal of amino acid sequence of the human serum albumin is substituted by alanine: H34

### Modified human serum albumin-thioredoxin fusion body

In this disclosure, a human serum albumin-thioredoxin fusion body means a fusion body in which a human serum albumin and a thioredoxin are fused or conjugated while keeping the action to prolong the thioredoxin's half-life in blood. According to an embodiment of the present invention, the human serum albumin and the thioredoxin are connected by means of a linker such as a polypeptide.

In addition, in the present invention, the thioredoxin is the modified body in which at least cysteine at position 73 from the N-terminal of the amino acid sequence thereof is substituted by another amino acid. The thioredoxin has a total of 5 cysteine residues at positions 32, 35, 62, 69, and 73 from the N-terminal of the sequence thereof. In the present invention, among these, at least cysteine at position 73 is substituted by another amino acid. According to the present invention, the amino acid that substitutes the cysteine is not particularly limited, but according to a preferred embodiment of the present invention, the cysteine is substituted by neutral amino acids, more preferably alanine, serine, or glycine. In the present invention, the most preferable substitution is by alanine. The substituting amino acids can be modified, for example, in some structural portions thereof.

According to a preferred embodiment of the present invention, the thioredoxin is a modified body in which in addition to substitution of cysteine at position 73, any one of or both the cysteine at positions 62 and 69 is/are substituted by another amino acid. In this embodiment, too, the amino acid that substitutes the cysteine is not particularly limited, but according to a preferred embodiment of the present invention, the cysteine is substituted by neutral amino acids, more preferably by alanine, serine, or glycine. In the present invention, the most preferable substitution is by alanine. However, because cysteines at positions 32 and 35 from the N-terminal of the thioredoxin is the active center of the oxidation reduction activity, it is preferable in the present invention that these are not substituted by another amino acid. The replacing amino acids may be modified, for example, in some structural portions thereof.

In addition, according to an embodiment of the present invention, cysteine in the human serum albumin can be substituted by another amino acid. The human serum albumin has cysteine at position 34 from the N-terminal of the amino acid sequence thereof, in which the cysteine can be substituted by another amino acid. In this embodiment, too, the amino acid that substitutes the cysteine is not particularly limited, but according to a preferred embodiment of the present invention, the cysteine is substituted by neutral amino acids, more preferably by alanine, serine, or glycine. In the present invention, the most preferable substitution is by alanine. The substituting amino acids can be modified, for example, in some structural portions thereof.

In addition, according to one embodiment of the present invention, in the amino acid sequences of the thioredoxin and human serum albumin, as long as the activities of them are not lost, one or two or more amino acids other than the cysteine residues can be substituted, deleted, or added. Therefore, according to one embodiment of the present invention, there is provided a modified body in which in the thioredoxin, cysteine that is located at a position equivalent to cysteine at position 73 from the N-terminal of the amino acid sequence thereof is substituted by another amino acid. In addition, according to another embodiment of the present invention, there is provided a modified body in which in the thioredoxin, cysteine that is located at a position equivalent to cysteine at position 62 and/or cysteine that is located at a position equivalent to cysteine at position 69 from the N-terminal of the amino acid sequence thereof are/is further substituted by another amino acid. In addition, the embodiment is provided in which in the serum albumin, cysteine that is located at a position equivalent to cysteine at position 34 from the N-terminal of the amino acid sequence thereof is substituted by another amino acid.

According to one preferred embodiment of the present invention, the modified human serum albumin-thioredoxin fusion body has the following amino acid sequence.

DAHKSEVAHRFKDLGEENFKALVLIAFAQYLQQCPFEDHVKLVNEVTEFAKTCVAD
ESAENCDKSLHTLFGDKLCTVATLRETYGEMADCCAKQEPERNECFLQHKDDNPN
LPRLVRPEVDVMCTAFHDNEETFLKKYLYEIARRHPYFYAPELLFFAKRYKAAFTEC
CQAADKAACLLPKLDELRDEGKASSAKQRLKCASLQKFGERAFKAWAVARLSQRFP
KAEFAEVSKLVTDLTKVHTECCHGDLLECADDRADLAKYICENQDSISSKLKECCE
KPLLEKSHCIAEVENDEMPADLPSLAADFVESKDVCKNYAEAKDVFLGMFLYEYAR
RHPDYSVVLLLRLAKTYETTLEKCCAAADPHECYAKVFDEFKPLVEEPQNLIKQNC
ELFEQLGEYKFQNALLVRYTKKVPQVSTPTLVEVSRNLGKVGSKCCKHPEAKRMPC
AEDYLSVVLNQLCVLHEKTPVSDRVTKCCTESLVNRRPCFSALEVDETYVPKEFNA
ETFTFHADICTLSEKERQIKKQTALVELVKHKPKATKEQLKAVMDDFAAFVEKCCK
ADDKETCFAEEGKKLVAASQAALGLggggsggggsVKQIESKTAFQEALDAAGDKLVV
VDFSATWCGPCKMIKPFFHSLSEKYSNVIFLEVDVDDCQDVASECEVKCMPTF
QFFKKGQKVGEFSGANKEKLEATINELV

In the sequence above, the uppercase letters indicate the HSA's amino acid sequence, the lowercase letters the linker's sequence, and the uppercase, bold letters the Trx's sequence. C indicates the 34 cysteine in HSA. C (bold letter) indicates the Trx's 62, 69, and 73 cysteines from the linker side.

In addition, according to another embodiment of the present invention, the numbers of the "human serum albumin" and the "thioredoxin" included in the modified human serum albumin-thioredoxin fusion body are not only one for each, but can also be plural for each. Also, the bonding sites thereof are not particularly limited as long as they are bonded while keeping the prolonging action of the thioredoxin's half-life in blood. Illustrative examples of the modified body include HSA-Trx having the Trx linked at the C-terminal of HSA, Trx-HSA linked at the N-terminal, Trx-HSA-Trx linked at both the C-terminal and N-terminal, HSA-Trx-Trx having 2 Trx linked at the C-terminal, as well as HSA-HSA-Trx, Trx-HSA-HSA, Trx-HSA-HSA-Trx, and HSA-HSA-Trx-Trx in which those having 2 HSA linked are bonded to Trx.

According to the present invention, the type and length of the linker that connects "human serum albumin" and "thioredoxin" are not particularly limited, but the body connected by a polypeptide as mentioned above is preferred, and a preferable length thereof is 0 to about 40 amino acid residues.

### Preparation of modified human serum albumin-thioredoxin fusion body

The modified human serum albumin-thioredoxin fusion body according to the present invention may be prepared by known methods. For example, using the albumin fusion technique described in PTL 1, the human serum albumin is fused with the thioredoxin. In this process, by changing cysteine codon in each sequence to the codon of another amino acid, the modified body can be obtained.

### Use of modified human serum albumin-thioredoxin fusion body

The modified human serum albumin-thioredoxin fusion body according to the present invention can improve the activity of the thioredoxin itself by modification, and in addition, has an advantage that the activity thereof can be expressed stably. Furthermore, the modified human serum albumin-thioredoxin fusion body according to the present invention is increased in its safety because immunogenicity thereof is decreased. In particular, when compared with the non-modified serum albumin-thioredoxin fusion body, the modified serum albumin-thioredoxin fusion body according to the present invention is advantageous in that it does not form the aggregate, which is presumably a dimer thereof, and that antibody induction, which is presumably a side reaction thereof, is reduced, and moreover, the antibody is hardly observed. The reduction in the antibody induction will result in non-occurrence of the anaphylactic reaction.

Because of the advantageous effects mentioned above, the modified serum albumin-thioredoxin fusion body according to the present invention is expected to be used in the uses utilizing the heretofore known thioredoxin activities, especially in the uses in which heretofore known preventive or therapeutic effects are expected; in addition, this may be used for prevention or treatment of the diseases whose therapeutic effects will be made clear in future. Illustrative examples of the disease to which this can be used include the diseases that can be cured by pharmacological activities such as an anti-oxidative action, activity control of a cytokine transcription factor, an anti-apoptotic action, and an anti-inflammatory action (diseases such as alcoholic liver injury, non-alcoholic liver injury, acetaminophen hepatitis, fulminant hepatitis, liver cirrhosis, acute kidney injury, chronic kidney injury, cisplatin kidney disease, rhabdomyolysis, contrast agent kidney disease, chronic kidney disease, diabetes kidney disease, organ fibrosis, autoimmune disease, Alzheimer disease, Parkinson disease, peripheral neuropathy, cerebral infarction, arteriosclerosis, cardiac infarction, heart failure, cardiac infarction, acute respiratory distress syndrome, chronic obstructive pulmonary disease, allergic pneumonitis, influenza pneumonia, bronchial asthma, etc.).

Therefore, according to one embodiment of the present invention, provided are a pharmaceutical composition including the modified serum albumin-thioredoxin fusion body according to the present invention, and a use for production of a pharmaceutical composition of the modified serum albumin-thioredoxin fusion body according to the present invention. According to another embodiment of the present invention, provided are the pharmaceutical composition including the modified serum albumin-thioredoxin fusion body according to the present invention that can be used for prevention or treatment of the diseases curable by pharmacological activities such as an anti-oxidative action, an activity control of a cytokine transcription factor, an anti-apoptotic action, and an anti-inflammatory action, and the use of the modified serum albumin-thioredoxin fusion body according to the present invention.

### [Examples]

The present invention will be described in detail by Examples, but the present invention is not limited by these Examples.

### Example 1: Activities of modified bodies having cysteine replaced

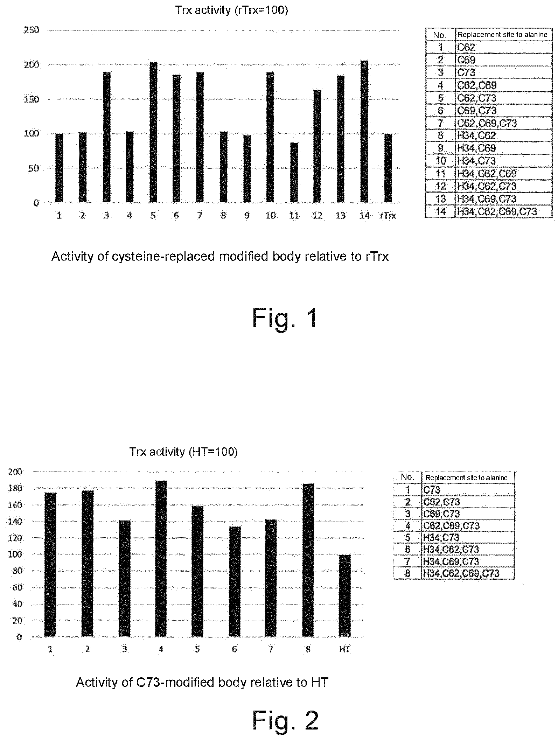

Modified HSA-Trx each was prepared in which one, two, three, or all (4) of the 34 cysteine in the HSA sequence, and the 62, 69, and 73 cysteines in the Trx sequence in the HSA-Trx was or were substituted by alanine. In the CHO cell expression plasmid of HSA-Trx, the plasmid having the above-mentioned sequence encoding the cysteine changed to the codon encoding alanine was constructed. By transfecting the plasmid into a CHO cell, the culture supernatant in which the modified HSA-Trx was produced was obtained. Transfection and cultivation were carried out using the ExpiCHO Expression System (ThermoFisher) in accordance with the manual thereof. The modified HSA-Trx was purified from the resulting culture supernatant by using Capture Select Human Albumin Affinity Matrix (Thermo Scientific) and Capto MMC (GE Healthcare), and concentrated by using Amicon Ultra-15 30K (Millipore); then, the buffer thereof was changed to PBS. The Trx activities of them were measured using Thioredoxin assay kit (IMCO) and the manual thereof; then, the relative activities to the recombinant human Trx (rTrx) attached thereto were calculated (rTrx as 100%). The results are as shown in Fig. 1.

Relative to rTrx, the modified body replaced at C62 showed 100%, the modified body replaced at C69 showed 101%, the modified body at C73 showed 189%, the modified body replaced at C62 and C69 showed 102%, the modified body replaced at C62 and C73 showed 204%, the modified body replaced at C69 and C73 showed 186%, the modified body replaced at C62, C69 and C73 showed 189%, the modified body replaced at H34 and C62 showed 103%, the modified body replaced at H34 and C69 showed 97%, the modified body replaced at H34 and C73 showed 189%, the modified body replaced at H34, C62 and C69 showed 87%, the modified body replaced at H34, C62 and C73 showed 164%, the modified body replaced at H34, C69 and C73 showed 184%, and the modified body replaced at H34, C62, C69 and C73 showed 207%; therefore, they showed the same or even higher activities. In particular, all the modified bodies replaced at C73 showed high values of 164% to 207%. From the above results, it was found that the Trx activities resulted from substitution of H34, C62, C69, and C73 with alanine were same or even higher as compared with rTrx, and that the substitutions including C73 increased the activities.

### Example 2: Activities of C73-modified bodies relative to non-modified body (HT)

The Trx activities of the C73-modified bodies and of HT were measured, and the activities of the C73-modified bodies relative to HT were calculated (HT as 100%). The measurements were carried out in the same manner as Example 1. The results are as shown in Fig. 2.

Relative to HT, the modified body replaced at C73 showed 175%, the modified body replaced at C62 and C73 showed 177%, the modified body replaced at C69 and C73 showed 141 %, the modified body replaced at C62, C69 and C73 showed 189%, the modified body replaced at H34 and C73 showed 159%, the modified body replaced at H34, C62 and C73 showed 134%, the modified body replaced at H34, C69 and C73 showed 142%, and the modified body replaced at H34, C62, C69 and C73 showed 186%; thus, all the C73-modified bodies showed high activities of 134 to 189% relative to HT. Accordingly, it was found that substitution including the C73 cysteine can increase the Trx activity as compared with HT, in which no substitution is carried out.

### Example 3: Electrophoresis of modified bodies

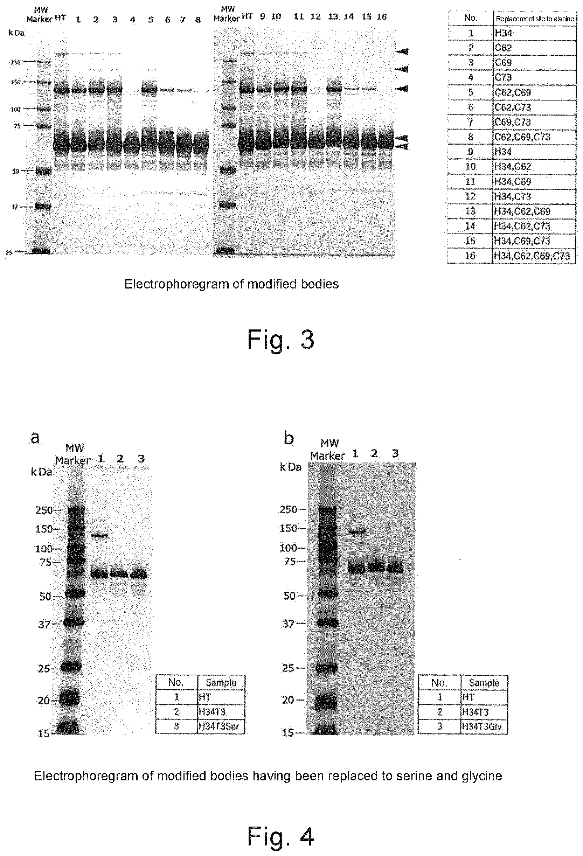

In order to clarify the molecular forms of the modified bodies in which cysteine is replaced, electrophoresis study was carried out. A sample 100 ng was mixed with the application solution (composed of sodium dodecylsulfate, glycerin, bromophenol blue, and distilled water; pH 6.8); and after the resulting mixture was boiled at 100°C for 3 minutes, this was applied to the 10% electrophoresis gel (ATTO) to carry out electrophoresis at 25 mA for about 55 minutes using the tricine electrophoresis buffer (composed of Tris, tricine, sodium dodecylsulfate, and distilled water; pH: approx. 8.6). After the gel was fixed for about 20 minutes using the fixing solution (composed of methanol, acetic acid, and distilled water), this was silver-stained using Silver Stain KANTO III (Tokyo Chemical Industry) and the manual thereof. The results are as shown in Fig. 3.

In this figure, the double arrow indicates the molecular size of the HSA-Trx, and the single arrow indicates the molecular sizes of the aggregates. In HT, which is the non-replaced body, in addition to the molecular size of the HSA-Trx, many aggregates are observed. In the replaced bodies at H34, C62, C69, and at C62 and C69, aggregates are observed, similarly to HT. On the contrary, the decreases in the aggregates were recognized in the C73-replaced bodies including substitutions at C73, at C62 and C73, at C69 and C73, at C62, C69 and C73, at H34 and C73, at H34, C62 and C73, at H34, C69 and C73, and at H34, C62, C69 and C73; and the aggregate's bands were not recognized in the replaced body at H34, C62, C69 and C73. From the above results, it became clear that the aggregates were reduced by the substitution of cysteine including C73, and that almost all of the aggregates were not recognized by the substitution of all the cysteines.

### Example 4: Modified bodies replaced to serine or glycine

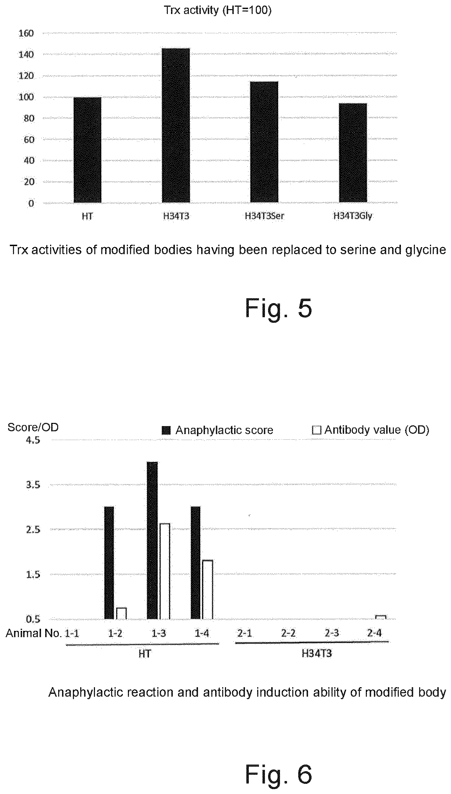

Using the method as same as Example 1, 4 cysteines at H34, C62, C69 and C73 were substituted by serine or glycine to obtain the modified HSA-Trx (hereinafter, the substitution with serine is abbreviated as H34T3Ser, with glycine as H34T3Gly, and with alanine as H34T3). The electrophoresis and measurement of Trx activity of these were carried out using the methods of Examples 1 and 2. The results are as shown in Figs. 4 and 5.

In Fig. 4, electrophoregram are shown for the modified body substituted by serine in a, and for the modified body substituted by glycine in b. In both the modified bodies, similarly to H34T3 the aggregate bands were not recognized, indicating that these substitutions resulted in reduction of the aggregates. Fig. 5 illustrates the measurement results of Trx activity relative to HT, showing 145% in H34T3, 115% in H34T3Ser, and 94% in H34T3Gly; thus, they are almost the same as or higher than HT. As the results of study on the Trx activity of the modified body having cysteine substituted by amino acid of alanine, serine, or glycine relative to the Trx activity of HSA-Trx, the substitution with alanine increased the activity most.

### Example 5: Safety of modified body

In order to confirm the safety of the modified body, whether or not the anaphylactic reaction and antibody induction occur by two administrations thereof to mice was studied.

Using ICR mice (6 weeks old male mice, Slc) with 4 mice in each group, comparison was performed between HT and the modified H34T3. From the tale vein, 0.1 µmol/kg of each HT and H34T3 was administered twice with 1 week interval. The anaphylactic reaction was evaluated in accordance with the scores as shown in Table 1 below until 120 minutes immediately after the second administration.

**Table 1**

| Anaphylactic reaction | Score |
|---|---|
| No change | 0 |
| Piloerection, nose-scratch, instable behavior, rapid breathing | 1 |
| Mastication, coughing, sneezing, crouching | 2 |
| Dyspnea, urination, defecation, listlessness, cyanosis | 3 |
| Convulsions, overturn | 4 |
| Death | 5 |

With regard to the antibody, a blood was partially taken out from the tale vein at 6 days after the first administration; and after subjected to a blood solidification reaction at 37°C for 30 minutes, this was centrifuged at 3500 rpm for 10 minutes to obtain a serum sample. The serum sample was kept at -80°C until ELISA measurement. The ELISA measurement was performed in accordance with the following method. The concentration of H34T3 in the solidification solution (0.1 mol/L Carbonate Buffer: pH9.6) was adjusted at 1 µg/mL; then, this was added to the ELISA plate with the amount of 100 µL/well and allowed to be statically left for one overnight at 4°C for natural adsorption to make this a solid phase. After washed with 0.05% Tween20 in PBS (hereinafter PBST) three times, a blocking solution (original Block Ace (KAC) solution was diluted by PBST to 1/4 concentration) was added with the amount of 250 µL/well; then, the reaction was carried out at room temperature for 2 hours. After washed with PBST three times, the serum sample was diluted with PBST/Block Ace solution (original Block Ace (KAC) solution was diluted by PBST to 1/10 concentration) to 1/200; then this was added with the amount of 100 µL/well to carry out the reaction at 37°C for 2 hours. After washed with PBST three times, the solution having an anti-Mouse-IgG-HRP (ZyMax, 81-6720) diluted with the PBST/BlockAce solution to 1/2000 was added with the amount of 100 µL/well to carried out the reaction at 37°C for 1.5 hours. After washed with PBST twice and with distilled water twice, TMB+ (Dako) was applied with the amount of 100 µL/well to carry out the reaction at room temperature for 30 minutes. The reaction was terminated by adding 1-N sulfuric acid with the amount of 100µL/well; then, the absorbance (optical density: OD value) at 450 to 650 nm was measured.

The results are as shown in Fig. 6. In HT, 3 examples in 4 examples, medium degree or higher (serious from score 3) of the anaphylactic reaction took place; and the antibody induction was clearly recognized in 2 examples. In H34T3, the anaphylactic reaction was not recognized, and the antibody induction was hardly recognized. From the above results, it can be said that the modified body having its cysteine substituted by the amino acid has increased its safety to immunological responses.

### Example 6: Effectiveness of modified bodies to liver diseases

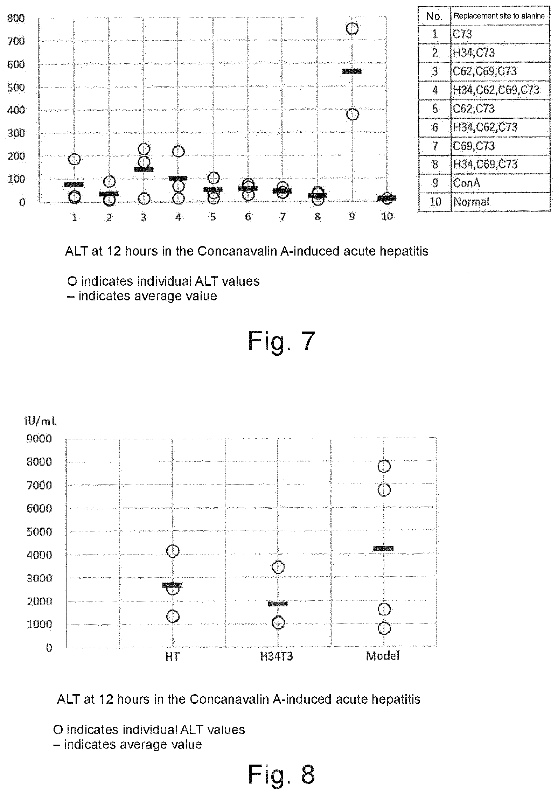

The effectiveness of the modified bodies was confirmed using the hepatitis induction model by Concanavalin A, then, HT and the H34T3 modified body were compared. The study was carried out using the C57BL/6 mice with 2 to 4 mice in each group. In the study of the effectiveness of the modified bodies the sample was administered from the tale vein with the amount of 0.05 µmol/kg. In the comparison study between HT and H34T3, the administration amount from the tale vein was 0.4 µmol/kg. Between 5 to 15 minutes after the sample administration, 12 mg/kg of Concanavalin A (WAKO) was administered from the tale vein. At 12 hours after administration of Concanavalin A, using Transaminase CII Test Wako (FUJIFILM), the alanine aminotransferase (ALT) was measured in accordance with the manual thereof. As a result, by administration of Concanavalin A, the increase in ALT was recognized. In the study of the effectiveness of the modified body, versus 565 IU/mL as the ALT value of the model (14 IU/mL in the normal mouse (n=1), the modified body replaced at C73 showed 79 IU/mL, the modified body replaced at H34 and C73 showed 37 IU/mL, the modified body replaced at C62, C69 and C73 showed 141 IU/mL, the modified body replaced at H34, C62, C69 and C73 showed 102 IU/mL, the modified body replaced at C62 and C73 showed 54 IU/mL, the modified body replaced at H34, C62 and C73 showed 57 IU/mL, the modified body replaced at C69 and C73 showed 47 IU/mL, and the modified body replaced at H34, C69 and C73 showed 28 IU/mL; thus, these suppressed the hepatitis induction (Fig. 7). In the comparison study of HT and H34T3, versus 4241 IU/mL as the ALT value of the model, HT showed 2681 IU/mL, and H34T3 showed 1858 IU/mL; thus, both HT and H34T3 suppressed the hepatitis induction, whereas H34T3 suppressed more (Fig. 8). From the above results, it was found that the modified bodies having the cysteine including C73 replaced could suppress the hepatitis induction due to Concanavalin A, and that the modified bodies suppressed more strongly as compared with the non-modified body.

### Example 7: Effectiveness of modified body to kidney diseases (part 1)

The evaluation was carried out using the cisplatin-induced acute kidney injury (AKI) model. Using ICR mice (6 weeks old male mice, Sic) with 3 mice in each group, comparison was performed between HT and the modified H34T3. Each HT and H34T3 was administered with the amount of 0.25 µmol/kg from the tale vein, and then cisplatin (intravenous drip infusion 10 mg "Marco", Yakult) was administered from the tale vein with the amount of 15 mg/kg to induce AKI. At 96 hours after the AKI induction, the blood urea nitrogen (BUN) value was measured using Urea Nitrogen, Detection Kit, Colorimetric, DetectX (ArborAssays) and the manual thereof.

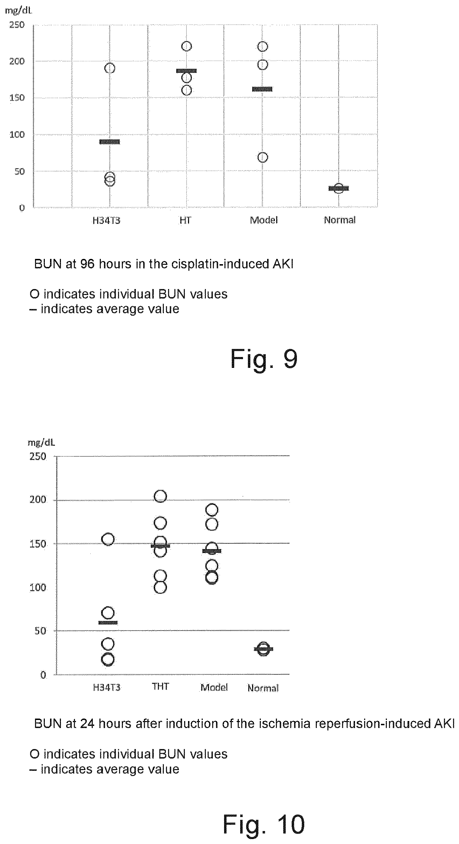

The results are as shown in Fig. 9. The increase in BUN was recognized by administration of cisplatin (in the figure, the average BUN in the model was 161 mg/dL, while the normal mouse (n=1) showed 26 mg/dL). The HT administration group showed 186 mg/dL, so that suppression effect of the AKI induction could not be recognized. The H34T3 administration group showed 90 mg/dL, so that suppression effect could be recognized. Accordingly, it can be concluded that the modified HSA-Trx having the cysteine substituted by another amino acid has the pharmacological effect to the kidney disease.

### Example 8: Effectiveness of modified body to kidney diseases (part 2)

The evaluation was carried out using the AKI model by ischemia reperfusion. Using C57BL/6 mice (7 or more weeks old male, Sic) with 2 to 3 mice in each group, comparison was performed between the non-modified THT (Trx is linked to the N-terminal of HT) and the modified H34T3. The right kidney was taken out while anesthetized at 1 week before the AKI induction and administration of the test substance. Each THT and H34T3 was administered from the tale vein with the amount of 0.1 µmol/kg. While anesthetized, the abdomen was opened, the left kidney was made ischemic; then, after 33 minutes, reperfusion was performed to induce the AKI. At 24 hours after the AKI induction, BUN was measured in the same way as Example 7. The study was carried out twice, and the results are summarized in Fig. 10.

By performing the ischemia reperfusion, versus 29 mg/dL as the BUN for the normal mouse, the increase in BUN to the average of 141 mg/dL (Model) was recognized. In the non-modified body THT, the BUN value was 147 mg/dL, so that the suppression effect on AKI was not recognized; but the H34T3 administration group showed 58 mg/dL, so that suppression effect was recognized. Accordingly, similarly to Example 7, it can be concluded that the modified HSA-Trx having the cysteine substituted by another amino acid has the pharmacological effect to the kidney disease.

### Example 9: Effectiveness of modified body to lung disease

The effect of the modified body to the acute pneumonia was studied using the bleomycin-induced mouse pulmonary fibrosis model. Using ICR mice (6 weeks old male mice, Sic) with 4 mice in each group, comparison was performed between non-modified HT and the modified H34T3. The pulmonary fibrosis was induced by administrating 1.2 mg/kg of bleomycin (Nippon Kayaku) into a trachea while anesthetized. At 1, 3, 5, and 8 days after administration of bleomycin, 0.4 µmol/kg of each HT and H34T3 was administered from the tale vein. The evaluation was carried out by measuring the left lung weight and hydroxyproline amount at 18 days after the bleomycin administration. The hydroxyproline amount was measured by the procedure described below. Into the sample, 1 mL of distilled water and 125 µL of 50 w/v% of TCA were added, and then the resulting mixture was homogenized, which was followed by allowing it to be statically left at 4°C for 20 minutes. The supernatant was removed by centrifugation with rotation of 10,000 rpm at 4°C for 10 minutes. After 500 µL of hydrochloric acid (Wako) was added, it was tapped so as to float the sample sediment, and then kept at 110°C for a whole day and night (about 18 hours). The dried sample sediment was suspended in 1 mL of distilled water. Solutions of L-hydroxyproline (Wako) as the standard substance with the concentrations of 0, 0.0156, 0.03125, 0.0625, 0.125, and 0.25 mg/mL were prepared to make a calibration curve. The sample and standard substance (200 µL) were dispensed into a tube, and then 500 µL of the chloramine T solution (composed of chloramine T, sodium acetate, 2-propanol, and distilled water) was added to it. After allowed to be statically left at room temperature for 20 minutes, 500 µL of the Ehnoh's reagent (composed of 4-(dimethylamino)benzaldehyde, 2-propanol, and perchloric acid) was added. After having been kept at 65°C for 15 minutes, 100 µL was distributed to a 96 well plate; then, the absorbance at 540 nm was measured.

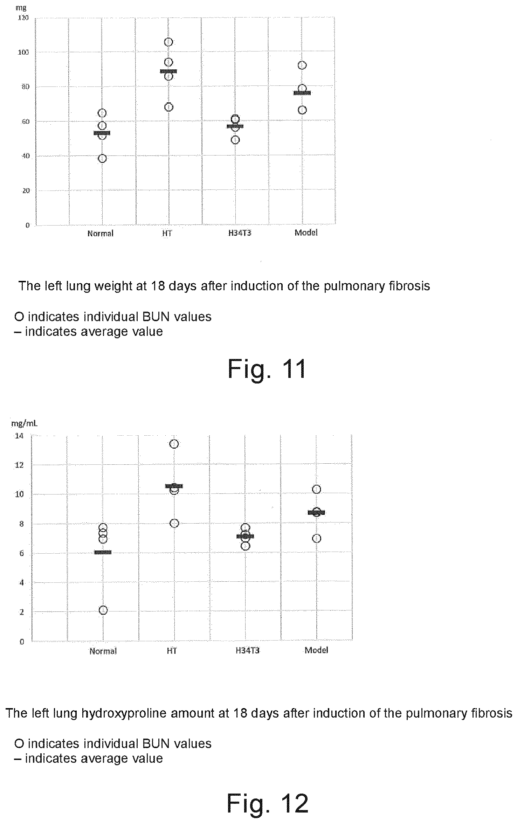

The results are as shown in Figs. 11 and 12. By induction of the pulmonary fibrosis due to administration of bleomycin, the lung weight was increased to 76 mg (Model group) versus the average of 53 mg in the normal mouse. There was no suppression effect in the HT administration group, which showed 89 mg, so that the weight was rather increased. On the other hand, the H34T3 administration group weighed 56.8 mg, so that the increase was hardly recognized. The hydroxyproline amount was 8.7 mg/mL in the model group versus 6.1 mg/mL in the normal mouse, so that the increase due to the fibrosis was recognized. There was no suppression effect in the HT administration group, which showed 10.5 mg/mL, so that the amount was rather increased. On the other hand, the amount in the H34T3 administration group was 7.1 mg/mL, so that the increase was hardly recognized. Accordingly, it can be concluded that the modified HSA-Trx having the cysteine substituted by another amino acid has the pharmacological effect to the acute pneumonia.

## Claims

1. A modified serum albumin-thioredoxin fusion body,
wherein the thioredoxin is a modified form in which at least cysteine at position 73 from N-terminal of an amino acid sequence thereof or located at a position equivalent thereto is substituted by another amino acid.

2. The modified serum albumin-thioredoxin fusion body according to claim 1, wherein the thioredoxin is a form in which cysteine at position 62 from N-terminal of the amino acid sequence thereof or located at a position equivalent thereto and/or cysteine at 69 position or located at a position equivalent thereto are/is further substituted by another amino acid.

3. The modified serum albumin-thioredoxin fusion body according to claim 1 or 2, wherein the serum albumin is a form in which cysteine at position 34 from N-terminal of the amino acid sequence thereof or located at a position equivalent thereto is substituted by another amino acid.

4. The modified serum albumin-thioredoxin fusion body according to any one of claims 1 to 3, wherein the amino acid that substitutes the cysteine is a neutral amino acid.

5. The modified serum albumin-thioredoxin fusion body according to any one of claims 1 to 4, wherein the amino acid that substitutes the cysteine is selected from alanine, serine, and glycine.

6. The modified serum albumin-thioredoxin fusion body according to any one of claims 1 to 5, wherein the amino acid that substitutes the cysteine is alanine.

7. The modified serum albumin-thioredoxin fusion body according to any one of claims 1 to 6, wherein
the thioredoxin is in the form cysteine at position 62 from N-terminal of the amino acid sequence thereof or located at a position equivalent thereto and/or cysteine at position 69 or located at a position equivalent thereto are/is further substituted by another amino acid, and
the serum albumin is in the form in which cysteine at position 34 from N-terminal of the amino acid sequence thereof or located at a position equivalent thereto is substituted by another amino acid.

8. The modified serum albumin-thioredoxin fusion body according to claim 7, wherein the amino acid that substitutes the cysteine is alanine.

9. The modified serum albumin-thioredoxin fusion body according to any one of claims 1 to 8, wherein the serum albumin and the thioredoxin are connected by a linker.

10. A pharmaceutical composition comprising the modified serum albumin-thioredoxin fusion body according to any one of claims 1 to 9.

11. The pharmaceutical composition according to claim 10, wherein the composition is for use in prevention or treatment of diseases curable by pharmacological activities of an anti-oxidative action, an activity control of a cytokine transcription factor, an anti-apoptotic action, and an anti-inflammatory action.

12. The pharmaceutical composition according to claim 11, wherein the diseases are alcoholic liver injury, non-alcoholic liver injury, acetaminophen hepatitis, fulminant hepatitis, liver cirrhosis, acute kidney injury, chronic kidney injury, cisplatin kidney disease, rhabdomyolysis, contrast agent kidney disease, chronic kidney disease, diabetes kidney disease, organ fibrosis, autoimmune disease, Alzheimer disease, Parkinson disease, peripheral neuropathy, cerebral infarction, arteriosclerosis, cardiac infarction, heart failure, cardiac infarction, acute respiratory distress syndrome, chronic obstructive pulmonary disease, allergic pneumonitis, influenza pneumonia, and bronchial asthma.

13. Use of the modified serum albumin-thioredoxin fusion body according to any one of claims 1 to 9 for production of a pharmaceutical composition.

14. Use of the modified serum albumin-thioredoxin fusion body according to any one of claims 1 to 9 for use in prevention or treatment of the diseases curable by pharmacological activities of an anti-oxidative action, an activity control of a cytokine transcription factor, an anti-apoptotic action, and an anti-inflammatory action,

15. The use according to claim 14 wherein the diseases are alcoholic liver injury, non-alcoholic liver injury, acetaminophen hepatitis, fulminant hepatitis, liver cirrhosis, acute kidney injury, chronic kidney injury, cisplatin kidney disease, rhabdomyolysis, contrast agent kidney disease, chronic kidney disease, diabetes kidney disease, organ fibrosis, autoimmune disease, Alzheimer disease, Parkinson disease, peripheral neuropathy, cerebral infarction, arteriosclerosis, cardiac infarction, heart failure, cardiac infarction, acute respiratory distress syndrome, chronic obstructive pulmonary disease, allergic pneumonitis, influenza pneumonia, and bronchial asthma.
